(19) Europäisches Patentamt
European Patent Office
Office européen des brevets

(11) Veröffentlichungsnummer: **0 062 834**
**A1**

(12) # EUROPÄISCHE PATENTANMELDUNG

(21) Anmeldenummer: **82102691.1**

(22) Anmeldetag: **31.03.82**

(51) Int. Cl.³: **C 07 C 155/02**
C 07 D 295/12, C 07 D 215/20
C 07 D 277/62, C 07 D 333/64
A 61 K 31/27, A 61 K 31/38
A 61 K 31/40, A 61 K 31/425
A 61 K 31/445, A 61 K 31/47

(30) Priorität: **03.04.81 DE 3113449**

(43) Veröffentlichungstag der Anmeldung:
**20.10.82 Patentblatt 82/42**

(84) Benannte Vertragsstaaten:
**CH DE FR GB IT LI**

(71) Anmelder: **HOECHST AKTIENGESELLSCHAFT**
**Postfach 80 03 20**
**D-6230 Frankfurt/Main 80(DE)**

(72) Erfinder: **Winkelmann, Erhardt, Dr.**
**Brunhildenweg 5**
**D-6233 Kelkheim (Tuanus)(DE)**

(72) Erfinder: **Dittmar, Walter, Dr.**
**Uhlandstrasse 5**
**D-6238 Hofheim am Taunus(DE)**

(72) Erfinder: **Raether, Wolfgang, Dr.**
**Falkensteinstrasse 6**
**D-6072 Dreieich(DE)**

(54) **Thiocarbaminsäureester, Verfahren zu ihrer Herstellung, sie enthaltende Arzneimittel und ihre Verwendung.**

(57) Es werden neue Thiocarbaminsäureester der allgemeinen Formel I

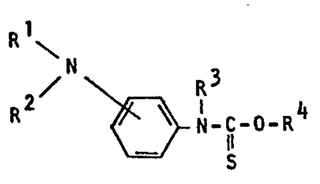

(1)

und ihre Salze mit physiologisch verträglichen Säuren und Verfahren zu ihrer Herstellung beschrieben.

Bevorzugt sind Verbindungen der allgemeinen Formel I, in welcher $R^1R^2N$- Dimethylamino oder Diäthylamino, vorzugsweise in 3-Position am Phenylring, $R^3$ Methyl und $R^4$ Äthylphenyl, Propylphenyl, tert.-Butylphenyl, Methylthio, vorzugsweise in 4-Position, oder Indanyl, 1,2,3,4-Tetrahydronaphthyl oder Benzthienyl, vorzugsweise in 6-Position mit dem Sauerstoff verbunden ist. Die neuen Verbindungen wirken gegen Pilzinfektionen der Haut. Sie können deshalb in entsprechenden Arzneimitteln verwendet werden.

EP 0 062 834 A1

Croydon Printing Company Ltd.

Thiocarbaminsäureester, Verfahren zu ihrer Herstellung, sie enthaltende Arzneimittel und ihre Verwendung

Aus der belgischen Patentschrift 627.322 sind Thiocarbaminsäureester mit fungistatischer Wirkung bekannt, insbesondere der N-Methyl-N-3-tolyl-thiocarbaminsäure-2-naphthylester (Tolnaftat) zur Bekämpfung von Pilzinfektionen der Haut.

Gegenstand der Erfindung sind Thiocarbaminsäureester der allgemeinen Formel I

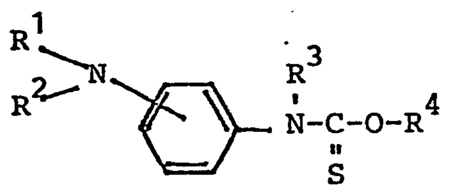

(I)

und ihre Salze mit physiologisch verträglichen Säuren, worin

$R^1$ und $R^2$ gleich oder verschieden sind und Wasserstoff, geradkettiges oder verzweigtes Alkyl mit ein bis drei Kohlenstoffatomen, wie Methyl, Äthyl, Propyl, Isopropyl, oder $R^1$ und $R^2$ zusammen eine Alkylenkette mit vier oder fünf Kohlenstoffatomen, die mit dem Stickstoffatom einen fünf- oder sechsgliedrigen heterocyclischen Ring bilden, der ein weiteres Heteroatom aus der Gruppe Stickstoff, Sauerstoff und Schwefel enthalten kann und seinerseits durch Methyl substituiert sein kann, wie Pyrrolidin, 2-Methyl-pyrrolidin, Piperidin, 2-Methyl-piperidin, Morpholin, 2-Methyl-morpholin, Piperazin, N-Methyl-piperazin oder Thiomorpholin bedeuten, wobei sich der Rest $R^1R^2N$- bevorzugt in 3- oder 4-Position befindet,

$R^3$ Methyl oder Äthyl,

$R^4$ - bevorzugt in 4-Position - substituiertes Phenyl, wobei der Substituent Alkyl, geradkettig, verzweigt mit 1 bis 6 Kohlenstoffatomen, wie Methyl, Äthyl, Propyl, Isopropyl, Butyl, Isobutyl, sek.-Butyl, tert.-Butyl, Amyl, Isoamyl, Hexyl, Isohexyl, oder cyclisch wie Cyclopentyl, Cyclohexyl, substituiert Alkyl mit 1 - 2 Kohlenstoffatomen, wie Methoxymethyl, Methylthiomethyl, Aminomethyl, N-Dimethylaminomethyl, N-Diäthylaminomethyl oder Pyrrolidinomethyl, Piperidinomethyl, Morpholinomethyl, N-Methylpiperazinomethyl, oder Alkoxy mit 1 - 2 Kohlenstoffatomen, wie Methoxy, Äthoxy, Alkylthio mit 1 - 2 Kohlenstoffatomen, wie Methylthio, Äthylthio, Alkylsulfinyl mit 1 - 2 Kohlenstoffatomen, wie Methylsulfinyl, Äthylsulfinyl, Alkylsulfonyl mit 1 - 2 Kohlenstoffatomen, wie Methylsulfonyl, Äthylsulfonyl, Formyl, Alkylcarbonyl mit mit 1 - 4 Kohlenstoffatomen, wie Acetyl, Propionyl und gegebenenfalls substituierte Oxime, wie Acetoxim, Acetyl-N-Dimethylaminoäthoxim, Halogen, wie Fluor, Chlor, Brom oder Jod, Trifluormethyl, Cyano, Carbamoyl, Thiocarbamoyl, Thiocyano, Isothiocyano, Nitro, Amino, Dialkylamino, wie Dimethylamino, Diäthylamino, 1 oder 2-Naphthyl, Indanyl, 1,2,3,4-Tetrahydronaphthyl, 1,2,3,4-Tetrahydro-1,4-endomethylen-, -1,4-endoäthylen-naphthyl, Indanon-1-yl, Tetralon-1-yl, Chinolyl, Isochinolyl, Benzthienyl, Benthiazolyl, - wobei die Bicyclen vorzugsweise in 6-Position mit dem Sauerstoff verbunden sind - bedeutet.

Bevorzugt sind Verbindungen der allgemeinen Formel I, in welcher $R^1R^2N$- Dimethylamino oder Diäthylamino, vorzugsweise in 3-Position am Phenylring, $R^3$ Methyl und $R^4$ Äthylphenyl, Propylphenyl, tert.-Butylphenyl, Methylthio, vorzugsweise in 4-Position, oder Indanyl, 1,2,3,4-Tetrahydronaphthyl oder Benzthienyl, vorzugsweise in 6-Position mit dem Sauerstoff verbunden ist.

Die Erfindung betrifft auch ein Verfahren zur Herstellung der Thiocarbaminsäureester der allgemeinen Formel I, dadurch gekennzeichnet, daß man

a) ein Anilin der allgemeinen Formel II

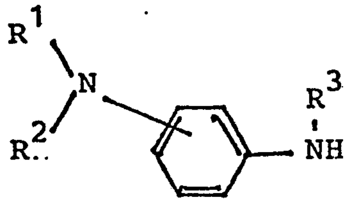

(II)

in der $R^1$, $R^2$, $R^3$ die oben angegebenen Bedeutungen haben, mit einer Thiokohlensäureesterverbindung der allgemeinen Formel III

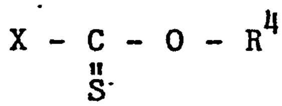

(III)

in der $R^4$ die oben angebenene Bedeutung hat und X Halogen, vorzugsweise Chlor, $C_1$ bis $C_4$-Alkylthio, vorzugsweise Methylthio, oder Phenylthio oder p-Tolylthio bedeutet, oder

b) eine Thiocarbaminsäureverbindung der allgemeinen Formel IV

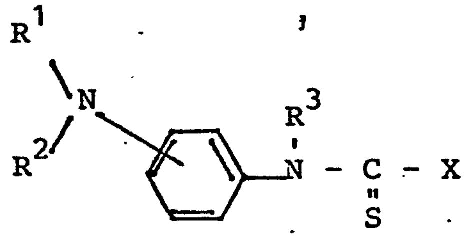

(IV)

in der $R^1$, $R^2$, $R^3$, X die oben angegebenen Bedeutungen haben, mit einem Phenolderivat der allgemeinen Formel V

$$HO - R^4 \qquad (V)$$

in der $R^4$ die oben angegebene Bedeutung hat, oder mit einem entsprechenden Alkali- oder Ammonium-phenolat, umsetzt.

Die Anilinderivate der Formel II sind bekannt.

Im Verfahren gemäß der Erfindung kommen als Ausgangsstoffe beispielsweise in Frage:
3-Amino-, 3-Methylamino-, 3-Äthylamino,- 3-Dimethylamino-, 3-Diäthylamino-, 3-Methyläthylamino-, 3-Pyrrolidino-, 3-Piperidino-, 3-Morpholino-, 3-N-Methylpiperazino-, 4-Dimethylamino-, 4-Diäthylamino-, -N-methyl-anilin oder -N-äthyl-

0062834

anilin.

Die Verbindungen der Formel IV können aus den eben aufgeführten substituierten Anilinen der Formel II durch Umsetzung mit Thiophosgen dargestellt werden.

Die Phenolderivate der Formel V sind bekannt. Als Ausgangsstoffe kommen erfindungsgemäß beispielsweise folgende Phenole in Frage die in 4-Position substituiert sind: Methyl, Äthyl, Propyl, Isopropyl, Butyl, Isobutyl, sek.-Butyl, tert.-Butyl, Amyl, Isoamyl, Cyclopentyl, Hexyl, Isohexyl, Cyclohexyl, Methoxymethyl, Methylthiomethyl, Aminomethyl, N-Dimethylaminomethyl, N-Diäthylaminomethyl, Pyrrolidinomethyl, Piperidinomethyl, Morpholinomethyl, N-Methylpiperazinomethyl, Methoxy, Äthoxy, Methylthio, Äthylthio, Methylsulfinyl, Äthylsulfinyl, Methylsulfonyl, Äthylsulfonyl, Formyl, Acetyl, Propionyl, Acetoxim, Acetyl-N-dimethylaminoäthoxim, Fluor, Chlor, Brom, Jod, Trifluormethyl, Cyano, Carbamoyl, Thiocarbamoyl, Thiocyano, Isothiocyano, Nitro, Amino, Dimethylamino, Diäthylamino.

Als Ausgangsstoffe der Formel V kommen ferner in Frage 1-Hydroxy-, 2-Hydroxy-naphthalin, 6-Hydroxy-indan, -1,2,3,4-tetrahydronaphthalin, -1,2,3,4-tetrahydro-1,4-endomethylen-naphthalin, -1,2,3,4-tetrahydro-1,4-endoäthylen-naphthalin, -indanon-1, -tetralon-1, -chinolin, -isochinolin, -benzthiophen, -benzthiazol.

Die Verbindungen der Formel III können aus den eben aufgeführten substituierten Phenolen der Formel V durch Umsetzung mit Thiophosgenen dargestellt werden.

Die Verfahren zur Herstellung der Verbindungen der allgemeinen Formel I nach Anspruch 2 werden zweckmäßigerweise wie folgt durchgeführt. Wobei das Verfahren a) bevorzugt ist.

Die Verbindungen der Formeln II und III, beziehungsweise der Formeln IV und V werden zweckmäßig in äquimolaren Mengen umgesetzt. Dabei empfiehlt sich die Verwendung eines Lösungs- oder Verteilungsmittels. Als Lösungsmittel kommen vorzugsweise in Frage Ketone wie Aceton, Methyläthylketon, Diäthylketon, Methylisobutylketon, Amide wie Dimethylformamid, Dimethylacetamid, N-Methylpyrrolidon, Tetramethylharnstoff, Hexamethylphosphorsäuretriamid, ferner Dimethylsulfoxid, Acetonitril oder Chloroform.

Die Reaktionszeiten betragen wenige Minuten bis einige Stunden. Die Reaktionstemperaturen liegen zwischen 0 und 100°C, vorteilhaft zwischen 60 und 80°C.

Bei den Umsetzungen der Reaktionspartner der Formel II und III, bzw. IV und V empfiehlt sich die Anwendung eines säurebindenden Mittels. Als säurebindende Mittel kommen beispielsweise in Frage Basen wie Triäthylamin, N-Äthylmorpholin oder Pyridin, Alkali-, Erdalkalicarbonate oder -bicarbonate, -hydroxide oder -alkoxide, z.B. Natriummethoxid. Anstelle der freien Phenolderivate können auch ihre Alkalimetall- oder Ammoniumsalze eingesetzt werden, insbesondere Natrium- oder Kaliumphenolat.

Die Isolierung der Verfahrenserzeugnisse erfolgt nach üblichen Methoden durch Abdestillieren der verwendeten Lösungsmittel oder Verdünnen der Reaktionslösung mit Wasser.

Gegebenenfalls kann eine Reinigung der Endprodukte durch Umkristallisieren aus einem geeigneten Lösungsmittel oder Lösungsmittelgemisch erfolgen.

Die Herstellung der Ausgangsstoffe nach der allgemeinen Formel III und IV erfolgt durch Umsetzung entsprechender Aniline der allgemeinen Formel II bzw. Phenolderivate der allgemeinen Formel Formel V mit Thiophosgen. Man setzt dabei äquimolare Mengen der beiden Reaktionspartner um, jedoch empfiehlt sich die Anwendung eines bis zu 10 %igen Überschußes an Thiophosgen. Die Umsetzung wird vorteilhaft in einem 2-Phasensystem, z.B. Chloroform/Wasser durchgeführt. Als chlorwasserstoffbindendes Mittel wird der wässrigen Phase eine äquivalente Menge Alkalihydroxid zugesetzt. Die Umsetzung erfolgt zweckmäßig bei 0 bis maximal +10°C. Die Isolierung erfolgt auf übliche Weise durch Destillation oder Kristallisation. In den meisten Fällen können die Rohprodukte der Umsetzung direkt weiterverarbeitet werden.

Die neuen Thiocarbaminsäureester gemäß der Erfindung besitzen eine dem eingangs genannten Tolnaftat überlegene Wirkung gegen Pilzinfektionen der Haut.

Sie sind in vitro sehr gut wirksam gegen Hautpilze, wie Trichophyton mentagrphytes und Microsporum canis oder Schimmelpilze, wie z.B. Aspergillus niger. Auch in vivo, z.B. bei der experimentellen Hautmykose am Meerschweinchen, sind die Verbindungen bei lokaler Anwendung sehr gut wirksam. Sie können deshalb zur Behandlung von Pilzerkrankungen bei Menschen und Tieren verwendet werden.

Als geeignete Anwendungsform der erfindungsgemäßen Verbindungen kommen beispielsweise in Frage Lösungen, Gelees, Puder, Cremes oder Salben, sowie Aerosole in Form von Spray.

Die Anwendungskonzentration in solchen Zubereitungen beträgt im allgemeinen 0,1 - 2 Gewichtsprozent.

Die Erfindung umfaßt auch pharmazeutische Zubereitungen, die eine Verbindung der allgemeinen Formel I und einen pharmazeutisch geeigneten Träger oder ein Verdünnungsmittel enthalten, sowie Kombinationen und Mischungen mit anderen gegen Pilze lokal wirksamen Stoffen.

Herstellungsbeispiele:

Beispiel 1:

Verfahren a

N-Methyl-N-(3-dimethylaminophenyl)-O-(4-tert.-butylphenyl)-thiocarbaminsäureester

Zu 15,0 g (0,1 Mol) 3-Dimethylamino-N-methylanilin, gelöst in 200 ml trockenem Aceton, werden 12,6 g (0,15 Mol) gepulvertes Natriumbicarbonat zugesetzt und dazu unter Rühren 22,86 g (0,1 Mol) Thiokohlensäure-(4-tert.-butylphenyl)-esterchlorid gelöst in 60 ml trockenem Aceton, zugetropft. Danach wird die Reaktionsmischung 30 Min. unter Rühren zum Rückfluß erhitzt. Die Reaktionsmischung wird am Rotationsverdampfer unter vermindertem Druck eingedampft, der Rückstand mit Methylenchlorid angerührt, abgesaugt, mit Methylenchlorid gewaschen, die vereinigten Methylenchloridlösungen über Natriumsulfat getrocknet und unter Zusatz von Kieselgel von farbigen Bestandteilen befreit. Die Methylenchloridlösung wird eingedampft und der Rückstand aus Petroläther umkristallisiert.
Man erhält so 26 g (77 % der Theorie) N-Methyl-N-(3-dimethylaminophenyl)-O-(4-tert.-butylphenyl)-thiocarbaminsäureester in Form von gelblichen Kristallen mit dem Schmelzpunkt 108°C.

Die Verbindung kann auf übliche Weise mittels alkoholischer Salzsäure in das Hydrochlorid (Fp. 201°C u. Zers.) überführt werden.

Das als Ausgangsstoff verwendete Thiokohlensäure-(4-tert.-butylphenyl)-esterchlorid wird nach bekannten Methoden durch Umsetzung von molaren Mengen Thiophosgen in Chloroform mit 4-tert.-Butyl-phenol in wässriger Natronlauge (Zweiphasensystem) bei 0 - 15°C dargestellt. Nach Abtrennen der Chloroformphase und Eindampfen wird das Rohprodukt durch Anrühren

mit Petroläther oder Leichtbenzin von restlichem Thiokohlensäure-bis-(4-tert.-butylphenyl)-ester befreit, abgesaugt, eingedampft und direkt weiter umgesetzt.

Dás so in sehr guter Ausbeute erhältliche Thiokohlensäure-(4-tert.-butylphenyl)-esterchlorid ist ein gelbliches Öl, welches gegebenenfalls destilliert werden kann. Es ist aber rein genug für weitere Umsetzungen.

Verfahren b)

N-Methyl-N-(3-dimethylaminophenyl)-O-(4-tert.-butylphenyl)-thiocarbaminsäureester wird durch Umsetzung von molaren Mengen N-Methyl-N-(3-dimethylaminophenyl)-thiocarbaminsäure-chlorid-Hydrochlorid mit 4-tert.-Butylphenol in Dimethylformamid in Gegenwart von Kaliumcarbonat dargestellt werden. Das Produkt schmilzt bei 107 - 108°C.

Das als Ausgangsstoff verwendete N-Methyl-N-(3-dimethylaminophenyl)-thiocarbaminsäurechlorid-Hydrochlorid erhält man nach bekannten Methoden durch Umsetzung von 3-Dimethylamino-N-methylanilin-Dihydrochlorid mit Thiophosgen.

Nach dem in Beispiel 1 beschriebenen und bevorzugten Verfahren a) können die in den folgenden Beispielen aufgeführten Verbindungen mit guten Ausbeuten dargestellt werden. Nach Verfahren b) werden die gleichen Verbindungen erhalten. Die Ausbeuten sind jedoch geringer.

Aus substituierten N-Methylanilinen und den Thiokohlensäureesterchloriden entsprechender Phenole bzw. Hydroxybicyclen erhält man

N-Methyl-N-(3-aminophenyl)-O-(4-tert.-butylphenyl)-thiocarbaminsäureester, Fp. 138° (Hydrochlorid Fp. 224°)
N-Methyl-N-(3-methylaminophenyl)-O-(4-tert.-butylphenyl)-thiocarbaminsäureester, Fp. 104°

N-Methyl-N-(3-äthylaminophenyl)-O-(4-tert.-butylphenyl)-thiocarbaminsäureester, Fp. 79°
eingedampft und direkt weiter umgesetzt.
N-Methyl-N-(3-dimethylaminophenyl)-O-(4-tert.-butylphenyl)-thiocarbaminsäureester, Fp. 108° (Hydrochlorid Fp. 200°)
N-Methyl-N-(3-diäthylaminophenyl)-O-(4-tert.-butylphenyl)-thiocarbaminsäureester, Fp. 198° Hydrochlorid
N-Methyl-N-(3-pyrrolidinophenyl)-O-(4-tert.-butylphenyl)-thiocarbaminsäureester, Fp. 157°
N-Methyl-N-(3-piperidinophenyl)-O-(4-tert.-butylphenyl)-thiocarbaminsäureester, Fp. 160°
N-Methyl-N-(3-morpholinophenyl)-O-(4-tert.-butylphenyl)-thiocarbaminsäureester, Fp. 189°
N-Methyl-N-(4-dimethylaminophenyl)-O-(4-tert.-butylphenyl)-thiocarbaminsäureester, Fp. 195°
N-Methyl-N-(4-diäthylaminophenyl)-O-(4-tert.-butylphenyl)-thiocarbaminsäureester, Fp. 142° Hydrochlorid
N-Methyl-N-(3-dimethylaminophenyl)-O-(4-methylphenyl)-thiocarbaminsäureester, Fp. 70°
N-Methyl-N-(3-dimethylaminophenyl)-O-(4-äthylphenyl)-thiocarbaminsäureester, Öl
N-Methyl-N-(3-dimethylaminophenyl)-O-(4-propylphenyl)-thiocarbaminsäureester, Öl
N-Methyl-N-(3-dimethylaminophenyl)-O-(4-N-dimethylaminomethylphenyl)-thiocarbaminsäureester, Öl
N-Methyl-N-(3-dimethylaminophenyl)-O-(4-methylthiophenyl)-thiocarbaminsäureester, Fp. 135° Hydrochlorid
N-Methyl-N-(3-dimethylaminophenyl)-O-(4-formylphenyl)-thiocarbaminsäureester, Öl
N-Methyl-N-(3-dimethylaminophenyl)-O-(4-acetylphenyl)-thiocarbaminsäureester, Fp. 204° Hydrochlorid
N-Methyl-N-(3-dimethylaminophenyl)-O-(4-chlorphenyl)-thiocarbaminsäureester, Fp. 96°
N-Methyl-N-(3-dimethylaminophenyl)-O-(4-trifluormethylphenyl)-thiocarbaminsäureester, Fp. 98°
N-Methyl-N-(3-dimethylaminophenyl)-O-(4-cyanophenyl)-thiocarbaminsäureester, Fp. 127°

N-Methyl-N-(3-dimethylaminophenyl)-O-(4-nitrophenyl)-thiocarbaminsäureester, Fp. 138°

N-Methyl-N-(3-dimethylaminophenyl)-O-(4-N-dimethylaminophenyl)-thiocarbaminsäureester, Fp. 102°

N-Methyl-N-(3-dimethylaminophenyl)-O-(naphthyl-2-)thiocarbaminsäureester, Fp. 104°

N-Methyl-N-(3-dimethylaminophenyl)-O-(indanyl-6)-thiocarbaminsäureester, Fp. 86°

N-Methyl-N-(3-dimethylaminophenyl)-O-(1,2,3,4-tetrahydronaphthyl-6)-thiocarbaminsäureester, Öl

N-Methyl-N-(3-dimethylaminophenyl)-O-(1,2,3,4-tetrahydro-1,4-endo-methylen-naphthyl-6)-thiocarbaminsäureester, Fp. 85°

N-Methyl-N-(3-dimethylaminophenyl)-O-(indanon-1-yl-6)-thiocarbaminsäureester, Fp. 117°

N-Methyl-N-(3-dimethylaminophenyl)-O-(tetralon-1-yl-6)-thiocarbaminsäureester, Fp. 84°

N-Methyl-N-(3-dimethylaminophenyl)-O-(chinolyl-6)-thiocarbaminsäureester, Fp. 95°

N-Methyl-N-(3-dimethylaminophenyl)-O-(benzthienyl-6)-thiocarbaminsäureester, Fp. 75°

N-Methyl-N-(3-dimethylaminophenyl)-O-(benzthiazolyl-6)-thiocarbaminsäureester, Fp. 116°.

Patentansprüche:

1. Thiocarbaminsäureester der allgemeinen Formel I

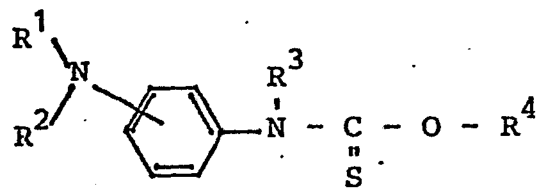 (I)

und ihre Salze mit physiologisch verträglichen Säuren, worin

$R^1$ und $R^2$ gleich oder verschieden sind und Wasserstoff, geradkettiges oder verzweigtes Alkyl mit ein bis drei Kohlenstoffatomen, wie Methyl, Äthyl, Propyl, Isopropyl, oder $R^1$ und $R^2$ zusammen eine Alkylenkette mit vier oder fünf Kohlenstoffatomen, die mit dem Stickstoffatom einen fünf- oder sechsgliedrigen heterocyclischen Ring bilden, der ein weiteres Heteroatom aus der Gruppe Stickstoff, Sauerstoff und Schwefel enthalten kann und seinerseits durch Methyl substituiert sein kann, wie Pyrrolidin, 2-Methyl-pyrrolidin, Piperidin, 2-Methyl-piperidin, Morpholin, 2-Methyl-morpholin, Piperazin, N-Methyl-piperazin oder Thiomorpholin bedeuten, wobei sich der Rest $R^1R^2N$- bevorzugt in 3- oder 4-Position befindet,

$R^3$ Methyl oder Äthyl,

$R^4$ - bevorzugt in 4-Position - substituiertes Phenyl, wobei der Substituent Alkyl, geradkettig, verzweigt mit 1 bis 6 Kohlenstoffatomen, wie Methyl, Äthyl, Propyl, Isopropyl, Butyl, Isobutyl, sek.-Butyl, tert.-Butyl, Amyl, Isoamyl, Hexyl, Isohexyl, oder cyclisch wie Cyclopentyl, Cyclohexyl, substituiert Alkyl mit 1 - 2 Kohlenstoffatomen, wie Methoxymethyl, Methylthiomethyl, Aminomethyl, N-Dimethylaminomethyl, N-Diäthylaminomethyl oder Pyrrolidinomethyl, Piperidinomethyl, Morpholinomethyl, N-Methylpiperazinomethyl, oder

Alkoxy mit 1 - 2 Kohlenstoffatomen, wie Methoxy, Äthoxy, Alkylthio mit 1 - 2 Kohlenstoffatomen, wie Methylthio, Äthylthio, Alkylsulfinyl mit 1 - 2 Kohlenstoffatomen, wie Methylsulfinyl, Äthylsulfinyl, Alkylsulfonyl mit 1 - 2 Kohlenstoffatomen, wie Methylsulfonyl, Äthylsulfonyl, Formyl, Alkylcarbonyl mit mit 1 - 4 Kohlenstoffatomen, wie Acetyl, Propionyl und gegebenenfalls substituierte Oxime, wie Acetoxim, Acetyl-N-Dimethylaminoäthoxim, Halogen, wie Fluor, Chlor, Brom oder Jod, Trifluormethyl, Cyano, Carbamoyl, Thiocarbamoyl, Thiocyano, Isothiocyano, Nitro, Amino, Dialkylamino, wie Dimethylamino, Diäthylamino, 1- oder 2-Naphthyl, Indanyl, 1,2,3,4-Tetrahydronaphthyl, 1,2,3,4-Tetrahydro-1,4-endomethylen-, -1,4-endoäthylen-naphthyl, Indanon-1-yl, Tetralon-1-yl, Chinolyl, Isochinolyl, Benzthienyl, Benthiazolyl, - wobei die Bicyclen vorzugsweise in 6-Position mit dem Sauerstoff verbunden sind - bedeutet.

2. Verbindungen der allgemeinen Formel I in Anspruch 1, in welcher $R^1R^2N$- Dimethylamino oder Diäthylamino, vorzugsweise in 3-Position am Phenylring, $R^3$ Methyl und $R^4$ Äthylphenyl, Propylphenyl, tert.-Butylphenyl, Methylthio, vorzugsweise in 4-Position, oder Indanyl, 1,2,3,4-Tetrahydronaphthyl oder Benzthienyl, vorzugsweise in 6-Position mit dem Sauerstoff verbunden ist, bedeuten.

3. Verfahren zur Herstellung der Thiocarbaminsäureester der allgemeinen Formel I, dadurch gekennzeichnet, daß man

a) ein Anilin der allgemeinen Formel II

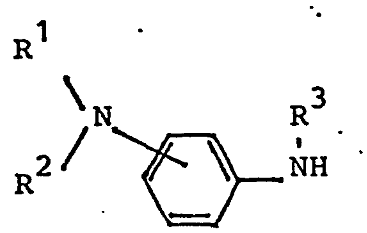

(II)

in der $R^1$, $R^2$, $R^3$ die oben angegebenen Bedeutungen haben, mit einer Thiokohlensäureesterverbindung der allgemeinen Formel III

$$X - \underset{S}{\underset{\|}{C}} - O - R^4 \qquad (III)$$

in der $R^4$ die oben angebenene Bedeutung hat und X Halogen, vorzugsweise Chlor, $C_1$ bis $C_4$-Alkylthio, vorzugsweise Methylthio, oder Phenylthio oder p-Tolylthio bedeutet, oder

b) eine Thiocarbaminsäureverbindung der allgemeinen Formel IV

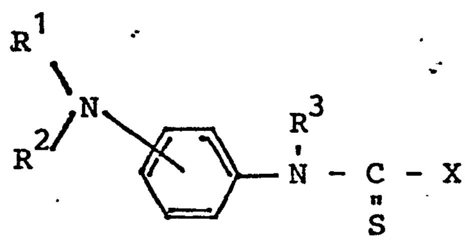

(IV)

in der $R^1$, $R^2$, $R^3$, X die oben angegebenen Bedeutungen haben, mit einem Phenolderivat der allgemeinen Formel V

$$HO - R^4 \qquad (V)$$

in der $R^4$ die oben angegebene Bedeutung hat, oder mit einem entsprechenden Alkali- oder Ammonium-phenolat, umsetzt.

4. Arzneimittel, gekennzeichnet durch einen Gehalt an einem Thiocarbaminsäureester der Formel I in Anspruch 1.

5. Verwendung von Thiocarbaminsäureester der Formel I in Anspruch 1 zur Behandlung von Pilzinfektionen.

**0062834**
Nummer der Anmeldung

Europäisches Patentamt

# EUROPÄISCHER RECHERCHENBERICHT

EP 82 10 2691.1

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | EP - A1 - 0 000 030 (BASF)<br>* Seiten 18 bis 22 *<br>-- | 1 | C 07 C 155/02<br>C 07 D 295/12<br>C 07 D 215/20<br>C 07 D 277/62<br>C 07 D 333/64<br>A 61 K 31/27<br>A 61 K 31/38<br>A 61 K 31/40<br>A 61 K 31/425<br>A 61 K 31/445<br>A 61 K 31/47 |
| P,A | DE - A1 - 3 018 670 (HOECHST)<br>* Ansprüche 1 bis 4 *<br>-- | 1,3-5 | |
| A | DE - A - 1 643 797 (CHEMISCHE FABRIK VON HEYDEN GMBH)<br>* Seiten 1, 2 *<br>-- | 1,4,5 | RECHERCHIERTE SACHGEBIETE (Int. Cl.³) |
| A | US - A - 3 288 673 (MOBIL OIL)<br>-- | | A 61 K 31/00<br>C 07 C 155/00<br>C 07 C 155/02<br>C 07 D 215/20<br>C 07 D 277/62<br>C 07 D 295/12<br>C 07 D 333/64 |
| A | US - A - 3 334 126 (NIPPON SODA KABUSHIKI KAISHA)<br>---- | | |
| | | | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 21-06-1982 | PHILLIPS |